# EUROPEAN PATENT APPLICATION

(11) **EP 4 427 827 A1**
(43) Date of publication of application: **11.09.2024**
(21) Application number: 23160830.8
(22) Date of filing: 08.03.2023
(51) Int. Cl.: B01D 3/14, B01D 3/38, B01D 3/40, C07C 7/08, C07C 15/46, C10G 7/08

(54) **A PLANT AND METHOD FOR SEPARATING AND PURIFYING STYRENE FROM A CRUDE MIXTURE COMPRISING STYRENE**

(71) Applicant: Sulzer Management AG, 8401 Winterthur (CH)
(72) Inventor: The designation of the inventor has not yet been filed
(74) Representative: Henkel & Partner mbB

(57) **Abstract**

A plant in particular for separating and purifying styrene from a crude mixture comprising styrene comprises a steam generating unit and a dividing-wall distillation column, wherein the dividing-wall distillation column comprises a dividing wall comprising a first portion extending at least essentially vertically downwards from a point being located below the top of the distillation column to a point being located above the bottom of the dividing-wall distillation column so as to subdivide the dividing-wall distillation column into a top section being located above the dividing-wall, into a bottom section being located below the dividing-wall and into a middle section comprising a first middle subsection being located on one side of the first portion of the dividing wall and a second middle subsection being located on the opposite side of the first portion of the dividing wall, wherein the dividing wall further comprises a second portion being connected with an upper part of the first portion of the dividing wall and extending into the second middle subsection to the inner wall of the dividing-wall distillation column, wherein at least a part of the bottom section has a smaller cross-sectional area than the middle section of the dividing-wall distillation column, wherein the dividing-wall distillation column further comprises an inlet line for feed, an inlet line for extractive solvent, an inlet line for steam being connected with the steam generating unit, an overhead outlet line, a bottom outlet line and a side outlet line being connected with the second middle subsection of the dividing-wall distillation column.

## Description

The present invention relates to a plant and method for separating and purifying styrene from a crude mixture comprising styrene.

Styrene is an important building block for polymers, such as polystyrene, acrylonitrile-butadiene-styrene/styrene-acrylonitrile resins, styrene-butadiene copolymer latexes, unsaturated polyester resins, styrene-butadiene rubber elastomers and latices. It is one of the highest volume commodity chemicals traded; over 30% of annual styrene production is traded internationally. Styrene is predominantly produced using benzene and ethylene as raw materials, wherein the benzene is alkylated with the ethylene to ethylbenzene, and wherein the ethylbenzene is converted via a dehydrogenation process or an ethylbenzene/styrene monomer process or via a propylene oxide/styrene monomer process, respectively, into styrene. Typically, styrene plants are located near ethylene crackers due to the gaseous nature of ethylene which makes it relatively difficult to transport as compared to benzene.

Apart from the on-purpose production route via the ethylbenzene/styrene monomer process or via the propylene oxide/styrene monomer process, styrene is also present in hydrocarbon streams, such as pyrolysis gasoline obtained from steam cracking of naphtha, the hydrocarbon fraction obtained from waste polystyrene (thermal or catalytic) pyrolysis, unsaturated gasoline obtained from various waste plastic (thermal or catalytic) pyrolysis etc. Styrene extraction from these hydrocarbon streams presents an attractive economic opportunity to the operator due to the low feedstock cost. The area of styrene production from waste polystyrene has especially gained mainstream attention due to the plastics recycling issues being encountered in the world presently. Waste plastics pose a serious threat to the environment. However, this separation is technically difficult due to the presence of close boiling molecules and impurities coming from the starting feedstock. For instance, during polystyrene recycling different sorts of waste polystyrene are fed into the catalytic or pyrolysis reactor. Due to the contamination of the waste plastic coupled with production of other close boiler compounds, like benzene, toluene, ethylbenzene, alpha-methyl styrene, cumene, n-propyl benzene etc., produced during the pyrolytic or catalytic step there is a need to have a reliable method to purify this styrene oil to the final ASTM grade styrene specification.

Separating and purifying styrene from a styrene containing crude mixture, such as one being produced with any of the aforementioned processes, is typically performed with one or more distillation steps and optionally together with a liquid/liquid extraction step. In some of these methods, an extractive distillation is used, in which the crude mixture is distilled in the presence of an extractive agent, which is typically a miscible, comparably high-boiling, relatively non-volatile component. The extractive agent changes the fugacities of appropriate components in the crude mixture, wherein the fugacity is the corrected partial vapor pressure in the mixture. Thereby, the separation of substances, which are only very difficult to separate by a regular distillation due to their close boiling points, is facilitated. These extractive distillation based purification processes are typically performed with two or three columns and are energy intensive as well as requiring a high capital expenditure (CAPEX). In order to decrease the required temperature for the extractive distillation, often steam is added as stripping agent into the distillation column in addition to the styrene containing crude mixture and in addition to the extractive agent. Of course, steam generation is an energy-intensive process.

Considering the above, the object underlying the present invention is to provide a plant for separating and purifying styrene from a crude mixture comprising styrene, which has during its operation only a low energy demand, which requires a low CAPEX and which allows to produce a very pure styrene composition.

In accordance with the present invention, this object is satisfied by providing a plant in particular for separating and purifying styrene from a crude mixture comprising styrene, wherein the plant comprises a steam generating unit and a dividing-wall distillation column, wherein the dividing-wall distillation column comprises a dividing wall comprising a first portion extending at least essentially vertically downwards from a point being located below the top of the distillation column to a point being located above the bottom of the dividing-wall distillation column so as to subdivide the dividing-wall distillation column into a top section being located above the dividing-wall, into a bottom section being located below the dividing-wall and into a middle section comprising a first middle subsection being located on one side of the first portion of the dividing wall and a second middle subsection being located on the opposite side of the first portion of the dividing wall, wherein the dividing wall further comprises a second portion being connected with an upper part of the first portion of the dividing wall and extending into the second middle subsection to the inner wall of the dividing-wall distillation column, wherein at least a part of the bottom section has a smaller cross-sectional area than the middle section of the dividing-wall distillation column, wherein the dividing-wall distillation column further comprises an inlet line for feed, an inlet line for extractive solvent, an inlet line for steam being connected with the steam generating unit, an overhead outlet line, a bottom outlet line and a side outlet line being connected with the second middle subsection of the dividing-wall distillation column.

This solution bases on the surprising finding that by separating and purifying styrene from a crude mixture comprising styrene in the aforementioned plant, which is in particular characterized in that a middle divided-wall distillation column, in which the dividing wall has a portion extending into the middle subsection towards the inner wall, thus closing this subsection of the middle subsection at least partially in the upward direction, is used and in that the dividing-wall distillation column has a bottom section, of which at least a part has a smaller cross-sectional area than the middle section of the dividing-wall distillation column, has a particular low energy demand and also has a particular low CAPEX. While the reason for the particular low energy demand is, among others, that the aforementioned plant has during its operation only a comparable low steam consumption, the reason for the particular low CAPEX is that the plant only requires one distillation column, namely the aforementioned dividing-wall distillation column. Moreover, the plant in accordance with the present invention only requires a minimum of plot space, all the more because a further distillation column for recovering the extractive agent from a mixture of extractive agent and styrene in addition to the aforementioned dividing-wall distillation column is not necessary. Thereby, the total distillation column height, i.e. the sum of the heights of all distillation columns of the plant, can be reduced. Moreover, the plant in accordance with the present invention surprisingly allows to produce a very pure styrene composition, namely a composition having a styrene content of 95 to 99% by weight, such as to at least 96.3% by weight.

In accordance with the present invention, the dividing wall of the dividing-wall distillation column comprises a first portion extending at least essentially vertically downwards from a point being located below the top of the distillation column to a point being located above the bottom of the dividing-wall distillation column so as to subdivide the dividing-wall distillation column into a top section being located above the dividing-wall, into a bottom section being located below the dividing-wall and into a middle section comprising a first middle subsection being located on one side of the first portion of the dividing wall and a second middle subsection being located on the opposite side of the first portion of the dividing wall. This means that the top section is the section, which is located - seen in the length direction of the dividing-wall distillation column, which is the vertical direction - above the uppermost point of the dividing wall, whereas the bottom section is the section being located below the lowermost point of the dividing wall and the middle section is the section being located between the top section and the bottom section. Thus, the dividing-wall distillation column consists - seen from its top to its bottom - of the top section, of the middle section and of the bottom section.

At least a part of the bottom section has a smaller cross-sectional area than the middle section of the dividing-wall distillation column, which means that - if the middle section has over its whole height the same (i.e. constant) cross-sectional area - at least a subsection of the bottom section has a smaller cross-sectional area than the cross-sectional area of the middle section of the dividing-wall distillation column. If the middle section has over its whole height not a constant, but a varying cross-sectional area, then at least a subsection of the bottom section has a smaller cross-sectional area than the average cross-sectional area of the middle section of the dividing-wall distillation column. If both, the middle section as well as the bottom section have over their whole height not a constant, but a varying cross-sectional area, then at least a subsection of the bottom section has a smaller average cross-sectional area than the average cross-sectional area of the middle section of the dividing-wall distillation column.

In accordance with the present invention, the first portion of the dividing wall of the dividing-wall distillation column extends at least essentially vertically downwards. Preferably, the angle between the first portion of the dividing wall and the length axis of the top dividing-wall distillation column is at most 20°, more preferably at most 10°, yet more preferably at most 5° and most preferably 0°. The length axis of the dividing-wall distillation column is the vertical axis. Moreover, it is preferred that the first portion of the dividing wall is a plate.

In a further development of the idea of the present invention, it is suggested that the first portion of the dividing wall extends - seen from the bottom to the top of the dividing-wall distillation column - from a point being located at 10 to 45% of the distance from the bottom to the top of the dividing-wall distillation column to a point being located at 50 to 90% of the distance from the bottom to the top of the dividing-wall distillation column. Even more preferably, the first portion of the dividing wall extends - seen from the bottom to the top of the dividing-wall distillation column - from a point being located at 25 to 40% of the distance from the bottom to the top of the dividing-wall distillation column to a point being located at 50 to 75% of the distance from the bottom to the top of the dividing-wall distillation column.

The first portion of the dividing-wall subdivides the middle section of the dividing-wall distillation column and preferably fluid tightly subdivides the middle section of the dividing-wall distillation column into a first and into a second middle subsection, which means that the first portion of the dividing-wall, which is preferably a plate, extends over the length-section of the dividing-wall distillation column, i.e. from a point at the inner wall of the dividing-wall distillation column to the opposite inner wall. Good results are in particular obtained, when the first portion of the dividing wall extends - seen from the bottom to the top of the dividing-wall distillation column - over 5 to 50%, more preferably over 10 to 40% and yet more preferably over 15 to 30% of the distance from the bottom to the top of the dividing-wall distillation column, i.e. of the length of the dividing-wall distillation column.

In accordance with a further preferred embodiment of the present invention, the first portion of the dividing wall subdivides the middle section of the dividing-wall distillation column, seen in cross-section of the dividing-wall distillation column, in about two equally sized subsections or halves, respectively. Therefore, it is preferred that one of the first and second middle subsections covers at least 30%, more preferably at least 40%, yet more preferably at least 45% and most preferably 50% of the total area of the cross-section of the middle section of the dividing-wall column, whereas the other of the first and second middle subsections covers the remainder to 100% of the total area of the cross-section of the middle section of the dividing-wall column. For instance, one subsection covers 43% and the other 57% of the total area of the cross-section or both subsections cover each exactly 50% of the total area of the cross-section. If the total area of the cross-section of the middle section varies over the axial length of the middle section, the above numeric values are related to the average total area of the cross-section of the middle section.

In accordance with the present invention, the second portion of the dividing wall extends into the second middle subsection to the inner wall of the dividing-wall distillation column. Preferably, the second portion of the dividing-wall is connected with an upper part of the first portion of the dividing wall and extends into the second middle subsection to the inner wall of the dividing-wall distillation column so as to at least partially close the top of the second middle subsection of the dividing-wall distillation column. Thereby, the top of the second middle subsection of the dividing-wall distillation column is at least partially closed, whereas the top of the first middle subsection is open. Preferably, the second portion of the dividing-wall is connected with one of its ends with the uppermost part of the first portion of the dividing wall and with its opposite end with the inner wall.

Even more preferably, the second portion of the dividing-wall is connected with an upper part (most preferably with the uppermost part) of the first portion of the dividing wall, extends into the second middle subsection to the inner wall of the dividing-wall distillation column and is connected with the inner wall of the dividing-wall distillation column so as to fluid tightly close the whole top of the second middle subsection of the dividing-wall distillation column, i.e. the second portion covers the whole top of the second middle subsection of the dividing-wall distillation column. In contrast to this, the top of the first middle subsection of the dividing-wall distillation is open, because it is not at all covered by an element, such as the second portion of the dividing wall. A fluid tightly closure of the whole top of the second middle subsection of the dividing-wall distillation column means that during the operation of the dividing-wall distillation column no ascending fluid (gas and/or liquid) may leave second middle subsection upwardly and that no descending fluid (gas and/or liquid) may enter the second middle subsection from above.

Preferably, both portions of the dividing wall are plates. Moreover, it is preferred that the first portion of the dividing wall comprises an upper edge and a lower edge and the second portion of the dividing wall comprises a first edge and a second edge, wherein the upper edge of the first portion of the dividing wall and the first edge of the second portion of the dividing wall are connected with each other over the whole length of both edges, and wherein the second edge of the second portion of the dividing wall is fluid tightly connected with the inner wall of the dividing-wall distillation column.

In a further development of the idea of the present patent application it is suggested that the second portion of the dividing-wall extends at least essentially horizontally to the inner wall of the dividing-wall distillation column, wherein the angle between the second portion of the dividing wall and the cross-sectional plane of the top dividing-wall distillation column is preferably at most 20°, more preferably at most 10°, still more preferably at most 5° and most preferably 0°.

In accordance with the present invention, at least a part of the bottom section of the dividing-wall distillation column has a smaller cross-sectional area than the middle section of the dividing-wall distillation column. Preferably, the cross-sectional area of the at least part of the bottom section of the dividing wall column is 10 to 99%, more preferably 20 to 95%, yet more preferably 30 to 90% and most preferably 40 to 60% of the cross-sectional area of the middle section. Moreover, it is preferred that the middle section has, seen over its length, i.e. along the vertical axis or length axis of the dividing-wall distillation column, respectively, an at least essentially constant cross-sectional area. Essentially constant cross-sectional area means in this connection that the difference between the maximal cross-sectional area and the minimal cross-sectional area along the vertical axis is less than 10%, preferably less than 5%, more preferably less than 1% and most preferably 0%. Moreover, it is preferred that the top section of the dividing-wall distillation column has, seen over its length, an essentially constant cross-sectional area, and/or that the middle section of the dividing-wall distillation column has, seen over its length, an essentially constant cross-sectional area. Furthermore, it is preferred that the top section of the dividing-wall distillation column has at least essentially the same cross-sectional area than the middle section, wherein essentially the same cross-sectional area means that the difference between the cross-sectional area of the top section and the cross-sectional area of the middle section is less than 10%, preferably less than 5%, more preferably less than 1% and most preferably 0%. If the cross-sectional area of the middle section and/or the cross-sectional area of the top section should be not constant over their lengths, then essentially the same cross-sectional area means that the difference between the average cross-sectional area of the top section and the average cross-sectional area of the middle section is less than 10%, preferably less than 5%, more preferably less than 1% and most preferably 0%. Again, if the cross-sectional area of the middle section and/or the cross-sectional area of the at least part of the of the bottom section of the dividing wall column should be not constant over their lengths, then the average cross-sectional area of the at least part of the bottom section of the dividing wall column is 10 to 99%, preferably 20 to 95%, more preferably 30 to 90% and most preferably 40 to 60% of the average cross-sectional area of the middle section. For instance, the (average) cross-sectional area of the smaller part of the bottom section is 30 to 70% and preferably 40 to 60% of the (average) cross-sectional area of the middle section of the dividing wall column. In an alternative embodiment, it is preferred that the (average) cross-sectional area of the smaller of the bottom section is 60 to 95% and preferably 70 to 85% of the (average) cross-sectional area of the middle section of the dividing wall column.

The bottom section of the dividing wall column may comprise, seen in the direction from the top to the bottom of the dividing-wall distillation column, a first upper subsection having a larger cross-sectional area and a second lower subsection having a smaller cross-sectional area. Preferably, the upper subsection has an at least essentially constant cross-sectional area over its length and also the lower subsection has an at least essentially constant cross-sectional area over its length. Furthermore, it is preferred that the cross-sectional area of the upper subsection of the bottom section is the same than the cross-sectional area of the middle section of the dividing-wall distillation column, whereas the cross-sectional area of the second subsection of the bottom section is 10 to 99%, preferably 20 to 95%, more preferably 30 to 90% and most preferably 40 to 60% of the cross-sectional area of the middle section.

The aforementioned embodiment may be modified in that the cross-sectional area of the first upper subsection of the bottom section is essentially the same than the cross-sectional area of the middle section, meaning that the difference between the cross-sectional area of the first upper subsection of the bottom section and the cross-sectional area of the middle section is less than 10%, preferably less than 5% and more preferably less than 1 %.

Moreover, it is possible, even if less preferred that the cross-sectional area of the first upper subsection of the bottom section varies over the length of the upper subsection and/or that the cross-sectional area of the second lower subsection of the bottom section varies over the length of the lower part and/or that the cross-sectional area of the middle section varies over the length of the middle section. The variation of the cross-sectional area over the length of the respective (subsection) may be up to 10% meaning that the difference between the maximal cross-sectional area and the minimal cross-sectional area along the vertical axis of the respective (sub)section may be up to 10%. In this case, it is preferred that the average cross-sectional area of the second subsection of the bottom section is 10 to 99%, preferably 20 to 95%, more preferably 30 to 90% and most preferably 40 to 60% of the average cross-sectional area of the middle section and that the average cross-sectional area of the first subsection of the bottom section is the same as the average cross-sectional area of the middle section.

In accordance with a particular preferred embodiment of the present invention, the bottom section of the dividing wall column comprises, seen in the direction from the top to the bottom of the dividing-wall distillation column, below the first subsection and below the second subsection a dome-shaped subsection forming the bottom. That means that the bottom section of the dividing wall column of this embodiment comprises and preferably consists, seen in the direction from the top to the bottom of the dividing-wall distillation column, of a first upper subsection, a second middle subsection and a third lower dome-shaped subsection forming the bottom, wherein the cross-sectional area of the first upper subsection of the bottom section is the same than the cross-sectional area of the middle section of the dividing-wall distillation column, whereas the cross-sectional area of the second middle subsection of the bottom section is 10 to 99%, preferably 20 to 95%, more preferably 30 to 90% and most preferably 40 to 60% of the cross-sectional area of the middle section. Preferably, each of the middle section, of the first upper subsection of the bottom section and of the second middle subsection of the bottom section have over its length a constant diameter. However, as described above it is also possible that the cross-sectional area of one, of two or of three of these (sub)sections varies over the length of the respective (sub)section, wherein the variation of the cross-sectional area over the length of the respective (sub)section may be up to 10%, preferably up to 5% and more preferably up to 1% meaning that the difference between the maximal cross-sectional area and the minimal cross-sectional area along the vertical axis of the respective parts of the bottom section may be up to 10%, preferably up to 5% and more preferably up to 1%. In this case, it is preferred that the average cross-sectional area of the first upper subsection of the bottom section is the same as the average cross-sectional area of the middle section and that the average cross-sectional area of the second middle subsection of the bottom section is 10 to 99%, preferably 20 to 95%, more preferably 30 to 90% and most preferably 40 to 60% of the average cross-sectional area of the middle section.

In accordance with an alternative particularly preferred embodiment of the present invention, the bottom section of the dividing wall column comprises, seen in the direction from the top to the bottom of the dividing-wall distillation column, below the first subsection and below the second subsection a third intermediate subsection and below the third intermediate subsection a dome-shaped subsection forming the bottom of the dividing-wall distillation column, wherein the second subsection tapers, seen in the direction from the top to the bottom of the second subsection, from the cross-sectional area of the first subsection to the cross-sectional area of the third intermediate subsection. That means that the bottom section of the dividing wall column of this embodiment comprises and preferably consists, seen in the direction from the top to the bottom of the dividing-wall distillation column, of a first upper subsection, a second middle subsection, a third intermediate subsection and a fourth lower dome-shaped subsection forming the bottom of the dividing-wall distillation column, wherein the cross-sectional area of the first upper subsection of the bottom section is preferably the same than the cross-sectional area of the middle section of the dividing-wall distillation column, whereas the cross-sectional area of the third intermediate subsection of the bottom section is 10 to 99%, preferably 20 to 95%, more preferably 30 to 90% and most preferably 40 to 60% of the cross-sectional area of the middle section. Preferably, each of the of the first upper subsection and of the third intermediate subsection of the bottom section have over its length a constant diameter. However, as described above it is also possible that the cross-sectional area of one, of two or of three of these (sub)sections varies over the length of the respective (sub)section, wherein the variation of the cross-sectional area over the length of the respective (sub)section may be up to 10%, preferably up to 5% and more preferably up to 1% meaning that the difference between the maximal cross-sectional area and the minimal cross-sectional area along the vertical axis of the respective parts of the bottom section may be up to 10%, preferably up to 5% and more preferably up to 1%. In this case, it is preferred that the average cross-sectional area of the first upper subsection of the bottom section is the same as the average cross-sectional area of the middle section and that the average cross-sectional area of the third intermediate subsection of the bottom section is 10 to 99%, preferably 20 to 95%, more preferably 30 to 90% and most preferably 40 to 60% of the average cross-sectional area of the middle section. The second middle subsection of this embodiment preferably tapers, seen in the direction from the top to the bottom, from the cross-sectional area of the first upper subsection to the cross-sectional area of the third intermediate subsection.

In a further development of the idea of the present invention, it is proposed that the dividing-wall distillation column has a circular cross-section, wherein the top of the top section of the dividing-wall distillation column is dome-shaped, and wherein the diameter of the subsection of the top section being below the dome-shaped top is over the length of the top section constant.

Good results are in particular obtained, when the inlet line for feed and the inlet line for extractive solvent are connected with the top section of the dividing-wall distillation column, wherein the inlet line for extractive solvent is connected with a location of the top section being above the location of the top section, with which the line for feed is connected, wherein the inlet line for steam is connected with the bottom section of the dividing-wall distillation column, and wherein the side outlet line is connected with the second middle subsection of the middle section of the dividing-wall distillation column.

Preferably, the side outlet line is connected with the second middle subsection of the dividing-wall distillation column.

The plant in accordance with the present invention may further comprise an overhead condenser and a liquid-liquid separator, wherein the overhead outlet line is connected with the overhead condenser and downstream thereof with the liquid-liquid separator. The liquid-liquid separator is preferably connected with a water line and is connected with a hydrocarbon line, wherein the water line leads into the steam generating unit and the hydrocarbon line splits into a discharge line and into a recycle line being connected with the top section of the dividing-wall distillation column.

In addition, it is preferred that the plant further comprises a side condenser and a liquid-liquid separator. While the side outlet line is connected with the side condenser and downstream thereof with the liquid-liquid separator, the liquid-liquid separator is connected with a water line and is connected with a hydrocarbon line, wherein the water line leads into the steam generating unit and the hydrocarbon line splits into a product line for pure styrene and into a recycle line being connected with the second middle subsection of the dividing-wall distillation column. Alternatively, the hydrocarbon line may be only a product line without splitting also into a recycle line. In this case, the water line may split into a line leading into the steam generating unit and into a recycle line being connected with the second middle subsection of the dividing-wall distillation column so that water is recycled from the liquid-liquid separator into the dividing-wall distillation column. Still alternatively, none of the hydrocarbon line and the water line may split into a recycle line. In the latter case, a line for feeding a slip stream may be connected with the dividing-wall distillation column.

In a further development of the idea of the present invention, it is suggested that the plant further comprises a reboiler, wherein the bottom section comprises an outlet line and in inlet line, both being connected with a recycle line leading from the outlet line to the inlet line, wherein the recycle line is connected with the reboiler.

For instance, in the aforementioned embodiment each of the outlet and of the inlet of the bottom section of the dividing-wall distillation column is arranged at a position being located, seen from the bottom to the top of the bottom section of the dividing-wall distillation column, between 50 and 99% and preferably between 75 and 95% of the distance from the lowermost to the uppermost subsection of the bottom section.

Good results are in particular obtained in this embodiment, when the dividing-wall distillation column has a circular cross-section and the bottom section comprises an upper subsection having the same diameter than the middle section and a lower subsection having a smaller diameter than the middle section, wherein each of the outlet and of the inlet of the bottom section of the dividing-wall distillation column is arranged in the upper subsection of the bottom section. A further subsection may be arranged between the upper subsection and the lower subsection, wherein the further subsection tapers from the cross-sectional area of the upper subsection to the cross-sectional area of the lower subsection.

In accordance with a further preferred embodiment of the present invention, a (further) reboiler may be arranged within the bottom section. It is preferred in this embodiment that the dividing-wall distillation column has a circular cross-section and the bottom section comprises an upper subsection having the same diameter than the middle section and a lower subsection having a smaller diameter than the middle section, wherein the reboiler is arranged within the lower subsection of the bottom section. A further subsection may be arranged between the upper subsection and the lower subsection, wherein the further subsection tapers from the cross-sectional area of the upper subsection to the cross-sectional area of the lower subsection. This embodiment allows a particular efficient and complete stripping of the crude mixture and requires only a minimum amount of internals, such as structured packings, in the column.

In accordance with an alternative preferred embodiment of the present invention, the subsection of the bottom section, which has a smaller cross-sectional area than the middle section of the dividing-wall distillation column, does not comprise a reboiler and is not connected with a line being connected with a reboiler. It is preferred in this embodiment that the whole bottom section does not contain any reboiler. This embodiment has, among others, the advantage of requiring only very low amounts of steam, of having a reduced CAPEX due to the omission of the reboiler and a reduced required height of the dividing-wall distillation column since the reboiler containing section is omitted.

In a further development of the idea of the present invention, it is proposed that the steam generating unit comprises a steam generator and a solvent regenerator. While the steam generator comprises an inlet being connected with a water line and an outlet for steam, the solvent regenerator comprises an inlet being connected with the outlet for steam of the steam generator, a further inlet line being connected with a solvent line being connected with the bottom outlet line of the dividing-wall distillation column and an outlet being connected with the inlet line for steam of the dividing-wall distillation column.

In accordance with a particular preferred embodiment of the present invention, the plant does not comprise any liquid-liquid extractor.

In a further aspect, the present invention relates to a method for separating and purifying styrene from a crude mixture comprising styrene comprising the steps of:
a) feeding a crude hydrocarbon composition containing styrene via the inlet line for feed, feeding an extractive solvent via the inlet line for extractive solvent and feeding an extractive solvent and water steam comprising mixture via the inlet line for steam into the dividing-wall distillation column of a plant in accordance with any of the preceding claims,
b) distilling the crude hydrocarbon composition, the extractive solvent and the water steam in the divided-wall column,
c) withdrawing a pure styrene composition via the side outlet line of the dividing-wall distillation column.

During the operation of the dividing-wall distillation column, a mixture comprising water steam and extractive solvent is fed via the inlet line for steam into the dividing-wall distillation column. However, for the ease of nomenclature this inlet line is shortly denoted in this application as inlet line for steam.

Preferably, the crude hydrocarbon composition contains 10 to 80% by weight, more preferably 30 to 60% by weight and most preferably 40 to 50% by weight of styrene. For instance, the crude hydrocarbon composition is a hydrocarbon C8 cut or a selectively hydrotreated hydrocarbon C8 cut of pyrolysis gasoline obtained from steam cracking of naphtha, the hydrocarbon fraction obtained from waste polystyrene (thermal or catalytic) pyrolysis, unsaturated gasoline obtained from various waste plastic (thermal or catalytic) pyrolysis etc.

Good results are in particular obtained, when the extractive solvent is a sulfone, such as a cyclic or acyclic sulfone, such as sulfolane.

In a further development of the idea of the present invention, it is suggested that the distillation is performed at a temperature of 40 to 140°C and at a pressure of 0.02 to 0.03 MPa and preferably at a temperature of 60 to 125°C and at a pressure of 0.005 to 0.15 MPa.

The ratio of extractive solvent based on the sum of extractive solvent and water steam in the mixture used in step a), which is generated in the steam generating unit, is preferably 80 to 95% by weight and more preferably 85 to 95% by weight. It is further preferred that the ratio of crude hydrocarbon composition based on the sum of crude hydrocarbon composition, extractive solvent and mixture comprising extractive solvent and water steam fed in step a) into the dividing-wall distillation column is 10 to 30% by weight and more preferably 15 to 20% by weight.

Subsequently, the present invention is described by means of illustrative, but not limiting figures, in which:
- Fig. 1: shows a schematic view of the plant for separating and purifying styrene from a crude mixture comprising styrene in accordance with one embodiment of the present invention.

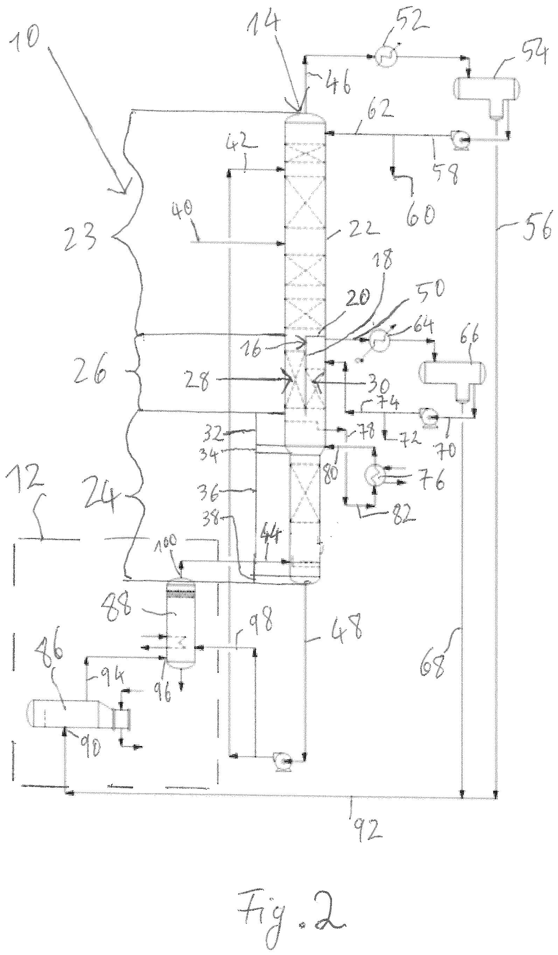

- Fig. 2: shows a schematic view of the plant for separating and purifying styrene from a crude mixture comprising styrene in accordance with an alternative embodiment of the present invention.

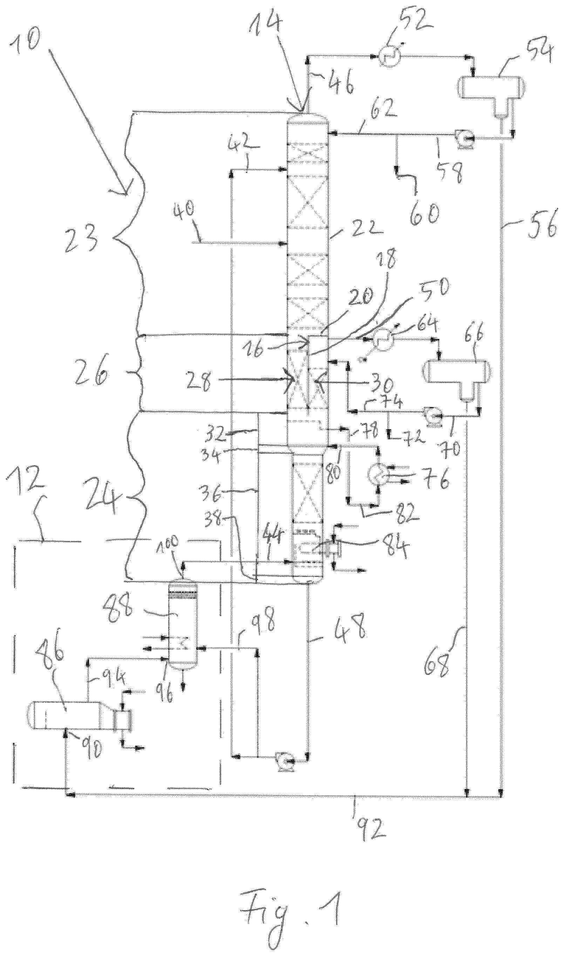

The plant 10 for separating and purifying styrene from a crude mixture comprising styrene shown in figure 1 comprises a steam generating unit 12 and a dividing-wall distillation column 14. The dividing-wall distillation column 14 comprises a dividing wall 16 comprising a first portion 18 extending vertically downwards and a second portion 20 being connected with the upper end of the first portion 18 and extending horizontally towards the inner wall 22, with which it is connected. The first portion 18 extends vertically downwards from a point being located below the top of the distillation column 14 to a point being located above the bottom of the dividing-wall distillation column 14 and thereby subdivides the dividing-wall distillation column 14 into a top section 23 being located above the dividing-wall 16, into a bottom section 24 being located below the dividing-wall 16 and into a middle section 26 comprising a first middle subsection 28 being located on one side of the first portion of the dividing wall and a second middle subsection 30 being located on the opposite side of the first portion 18 of the dividing wall 16. Both, the first middle subsection 28 and the second middle subsection 30 have the same cross-sectional area. The second portion 20 of the dividing wall 16, which is connected with the upper end of the first portion 18 and extends horizontally towards the inner wall 22, with which it is connected, fluid tightly closes the whole top of the second middle subsection 30 of the dividing-wall distillation column 14, i.e. the second portion 20 covers the whole top of the second middle subsection 30 of the dividing-wall distillation column 14. In contrast to this, the top of the first middle subsection 28 of the dividing-wall distillation 14 is open, because it is not at all covered by an element, such as the second portion 20 of the dividing wall 16. On account of the fluid tightly closure of the whole top of the second middle subsection 30 of the dividing-wall distillation column 14, no ascending fluid (gas and/or liquid) may leave second middle subsection 30 upwardly and no descending fluid (gas and/or liquid) may enter the second middle subsection 30 from above during the operation of the dividing-wall distillation column 14. Both, the top and the bottom of the dividing-wall distillation column 14 are dome-shaped.

The bottom section 24 of the dividing-wall distillation column 14 comprises, seen in the direction from the top to the bottom of the dividing-wall distillation column 14, a first subsection 32, a second subsection 34, a third subsection 36 and a fourth dome-shaped subsection 38 forming the bottom. The upper first subsection 32 of the bottom section 24 of the dividing-wall distillation column 14 has - seen along the length of the first subsection 32 - a constant cross-sectional area, which is the same as the constant cross-sectional area of the middle section 26 and the same as the cross-sectional area of the top section 23 below the dome-shaped top. However, the third subsection 36 of the bottom section 24 of the dividing-wall distillation column 14 has a constant cross-sectional area, which is smaller than the cross-sectional areas of the top section 23, of the middle section 26 and of the first subsection 32 of the bottom section 24. More specifically, the cross-sectional area of the third subsection 36 of the bottom section 24 of the dividing-wall distillation column 14 is about 50% of the cross-sectional area of the first subsection 32, i.e. the diameter of the third subsection 36 is about 70% of the first subsection 32. The second subsection 34 of the bottom section 24 of the dividing-wall distillation column 14 tapers along its length axis, seen from the top to the bottom, from the diameter of the first subsection 32 to the third subsection 36 of the bottom section 24.

The dividing-wall distillation column 14 further comprises an inlet line 40 for feed leading into the top section 23, an inlet line 42 for extractive solvent leading into the top section 23 at a position above the position where the inlet line 40 for feed leads into the top section 23, an inlet line 44 for steam being connected with the steam generating unit, an overhead outlet line 46, a bottom outlet line 48 and a side outlet line 50 being connected with the second middle subsection 30 of the middle section 26 of the dividing-wall distillation column 14. Furthermore, an overhead condenser 52 and a liquid-liquid separator 54 are provided, wherein the overhead outlet line 46 is connected with the overhead condenser 52 and downstream thereof with the liquid-liquid separator 54. The liquid-liquid separator 54 is connected with a water line 56 and is connected with a hydrocarbon line 58, wherein the water line 56 leads into the steam generating unit 12 and the hydrocarbon line 58 splits into a discharge line 60 and into a recycle line 62 being connected with the top section 23 of the dividing-wall distillation column 14. In addition, a side condenser 64 and a further liquid-liquid separator 66 are provided, wherein the side outlet line 50 is connected with the side condenser 64 and downstream thereof with the liquid-liquid separator 66. Also, the liquid-liquid separator 66 is connected with a water line 68 and is connected with a hydrocarbon line 70, wherein the water line 68 leads into the steam generating unit 12 and the hydrocarbon line 70 splits into a product line 72 for pure styrene and into a recycle line 74 being connected with the second middle subsection 30 of the middle section 26 of the dividing-wall distillation column 14. Furthermore, the plant 10 comprises a first reboiler 76, wherein the first subsection 32 of the bottom section 24 comprises an outlet line 78 and in inlet line 80, both being connected with a recycle line 82 leading from the outlet line 78 to the inlet line 80, wherein the recycle line 82 is connected with the first reboiler 76. A second reboiler 84 is arranged within the third subsection 36 of the bottom section 24 of the dividing-wall distillation column 14. This second reboiler 84 allows a particular efficient and complete stripping of the crude mixture and requires only a minimum amount of internals, such as structured packings, in the divided-wall distillation column 14.

Finally, the steam generating unit 12 comprises a steam generator 86 and a solvent regenerator 88. The steam generator 86 comprises an inlet 90 being connected with a water line 92, into which the two water lines 56, 68 lead, as well as an outlet line 94 for steam. The solvent regenerator 88 comprises an inlet 96 being connected with the outlet line 94 for steam of the steam generator 86, further comprises an inlet line 98 being connected with the solvent line 98 being connected with the bottom outlet line 48 of the dividing-wall distillation column 14 and further comprises an outlet 100 being connected with the inlet line 44 for steam of the dividing-wall distillation column 14.

During the operation, crude mixture containing styrene, for instance a C8 cut feed, is fed via the inlet line 40 into the dividing-wall distillation column 14, whereas extractive solvent is fed via the inlet line 42 and a mixture of (water) steam and extractive solvent is fed via the inlet line 44 into the dividing-wall distillation column 14. While the top section and the first middle subsection 28 function during the distillation as extractive distillation sections, the second middle subsection 30 functions during the distillation as rectification section and the bottom section 24 functions as solvent stripping section. The overhead stream comprising small amounts of non-aromatic hydrocarbons, of toluene, of mixed xylenes and of styrene as well as high amounts of water and ethylbenzene, is led through the overhead condenser 52 and liquid-liquid separator 54, in which the overhead stream is condensed and separated into an aqueous stream and into a hydrocarbon stream. While the aqueous stream is led via lines 56, 92 into the steam generator 86, the hydrocarbon stream is partly recycled via the recycle line 62 into the dividing-wall distillation column 14, whereas the rest thereof is withdrawn from the plant for instance as C8 raffinate via line 60. Via the bottom line 48, regenerated extractive solvent is withdrawn, which is partly led into the top section 23 of the dividing-wall distillation column 14 via the inlet line 42, whereas the rest thereof is led via the solvent line 98 into the solvent regenerator. The purified styrene stream is withdrawn via the side outlet line 50 from the second middle subsection 30 of the dividing-wall distillation column 14, and is condensed in the side condenser 64 and separated in the liquid-liquid separator 66 into an aqueous stream and into a hydrocarbon stream. While the aqueous stream is led via lines 68, 92 into the steam generator 86, the hydrocarbon stream is partly recycled via the recycle line 74 into the dividing-wall distillation column 14, whereas the rest thereof is withdrawn from the plant as pure styrene stream via the product line 72. The water is evaporated in the steam generator 86 into steam and then mixed in the solvent regenerator 88 with solvent, before the so produced mixture of water steam and solvent is fed via line 44 into the bottom section 24 of the dividing-wall distillation column 14.

The plant 10 for separating and purifying styrene from a crude mixture comprising styrene shown in figure 1

### Reference Numerals

- 10: Plant
- 12: Steam generating unit
- 14: Dividing-wall distillation column
- 16: Dividing-wall
- 18: First portion of dividing-wall
- 20: portion of dividing-wall
- 22: Inner wall
- 23: Top section of dividing-wall distillation column
- 24: Bottom section of dividing-wall distillation column
- 26: Middle section of dividing-wall distillation column
- 28: First middle subsection
- 30: Second middle subsection
- 32: First subsection
- 34: Second subsection
- 36: Third subsection
- 38: Fourth subsection
- 40: Inlet line for feed
- 42: Inlet line for extractive solvent
- 44: Inlet line for steam
- 46: Overhead outlet line
- 48: Bottom outlet line
- 50: Side outlet line
- 52: Overhead condenser
- 54: Liquid-liquid separator
- 56: Water line
- 58: Hydrocarbon line
- 60: Discharge line
- 62: Recycle line
- 64: Side condenser
- 66: Liquid-liquid separator
- 68: Water line
- 70: Hydrocarbon line
- 72: Product line for pure styrene
- 74: Recycle line
- 76: First reboiler
- 78: Outlet line
- 80: Inlet line
- 82: Recycle line
- 84: Second reboiler
- 86: Steam generator
- 88: Solvent regenerator
- 90: Inlet of steam generator
- 92: Water line
- 94: Outlet line for steam
- 96: Inlet of solvent regenerator
- 98: Solvent line
- 100: Outlet of solvent regenerator

## Claims

1. A plant in particular for separating and purifying styrene from a crude mixture comprising styrene, wherein the plant comprises a steam generating unit and a dividing-wall distillation column, wherein the dividing-wall distillation column comprises a dividing wall comprising a first portion extending at least essentially vertically downwards from a point being located below the top of the distillation column to a point being located above the bottom of the dividing-wall distillation column so as to subdivide the dividing-wall distillation column into a top section being located above the dividing-wall, into a bottom section being located below the dividing-wall and into a middle section comprising a first middle subsection being located on one side of the first portion of the dividing wall and a second middle subsection being located on the opposite side of the first portion of the dividing wall, wherein the dividing wall further comprises a second portion being connected with an upper part of the first portion of the dividing wall and extending into the second middle subsection to the inner wall of the dividing-wall distillation column, wherein at least a part of the bottom section has a smaller cross-sectional area than the middle section of the dividing-wall distillation column, wherein the dividing-wall distillation column further comprises an inlet line for feed, an inlet line for extractive solvent, an inlet line for steam being connected with the steam generating unit, an overhead outlet line, a bottom outlet line and a side outlet line being connected with the second middle subsection of the dividing-wall distillation column.

2. The plant in accordance with claim 1, wherein the angle between the first portion of the dividing wall and the length axis of the top dividing-wall distillation column is at most 20°, preferably at most 10°, more preferably at most 5° and most preferably 0°, wherein the first portion of the dividing wall extends, seen from the bottom to the top of the dividing-wall distillation column, from a point being located at 10 to 45% of the distance from the bottom to the top of the dividing-wall distillation column to a point being located at 50 to 90% of the distance from the bottom to the top of the dividing-wall distillation column and preferably from a point being located at 25 to 40% of the distance from the bottom to the top of the dividing-wall distillation column to a point being located at 50 to 75% of the distance from the bottom to the top of the dividing-wall distillation column.

3. The plant in accordance with claim 1 or 2, wherein one of the first and second middle subsections covers at least 30%, more preferably at least 40%, yet more preferably at least 45% and most preferably 50% of the total area of the cross-section of the middle section of the dividing-wall column, whereas the other of the first and second middle subsections covers the remainder to 100% of the total area of the cross-section of the middle section of the dividing-wall column.

4. The plant in accordance with any of the preceding claims, wherein the second portion of the dividing-wall is connected with an upper part of the first portion of the dividing wall, extends into the second middle subsection to the inner wall of the dividing-wall distillation column and is connected with the inner wall of the dividing-wall distillation column so as to fluid tightly close the top of the second middle subsection of the dividing-wall distillation column, wherein the second portion of the dividing-wall extends at least essentially horizontally to the inner wall of the dividing-wall distillation column, wherein the angle between the second portion of the dividing wall and the cross-sectional plane of the top dividing-wall distillation column is at most 20°, preferably at most 10°, more preferably at most 5° and most preferably 0°.

5. The plant in accordance with any of the preceding claims, wherein the cross-sectional area of the at least part of the bottom section of the dividing wall column is 10 to 99%, preferably 20 to 95%, more preferably 30 to 90% and most preferably 40 to 60% of the cross-sectional area of the middle section.

6. The plant in accordance with any of the preceding claims, wherein the bottom section of the dividing wall column comprises, seen in the direction from the top to the bottom of the dividing-wall distillation column, a first subsection and a second subsection, wherein the first subsection is above the second subsection, wherein the cross-sectional area of the upper subsection of the bottom section is the same than the cross-sectional area of the middle section of the dividing-wall distillation column, whereas the cross-sectional area of the second subsection of the bottom section is 10 to 99%, preferably 20 to 95%, more preferably 30 to 90% and most preferably 40 to 60% of the cross-sectional area of the middle section, wherein preferably the bottom section of the dividing wall column comprises, seen in the direction from the top to the bottom of the dividing-wall distillation column, below the first subsection and below the second subsection a dome-shaped subsection forming the bottom.

7. The plant in accordance with any of claims 1 to 5, wherein the bottom section of the dividing wall column comprises, seen in the direction from the top to the bottom of the dividing-wall distillation column, a first upper subsection, a second middle subsection, a third intermediate subsection and below the third intermediate subsection a dome-shaped subsection forming the bottom of the dividing-wall distillation column, wherein the cross-sectional area of the first upper subsection of the bottom section is the same than the cross-sectional area of the middle section of the dividing-wall distillation column, wherein the cross-sectional area of the third intermediate subsection of the bottom section is 10 to 99%, preferably 20 to 95%, more preferably 30 to 90% and most preferably 40 to 60% of the cross-sectional area of the middle section, and wherein the second middle subsection tapers, seen in the direction from the top to the bottom of the second middle subsection, from the cross-sectional area of the first upper subsection to the cross-sectional area of the third intermediate subsection, wherein preferably the dividing-wall distillation column has a circular cross-section, wherein the top of the top section of the dividing-wall distillation column is dome-shaped, and wherein the diameter of the subsection of the top section being below the dome-shaped top is over the length of the top section constant.

8. The plant in accordance with any of the preceding claims, wherein the inlet line for feed and the inlet line for extractive solvent are connected with the top section of the dividing-wall distillation column, wherein the inlet line for extractive solvent is connected with a location of the top section being above the location of the top section, with which the line for feed is connected, wherein the inlet line for steam is connected with the bottom section of the dividing-wall distillation column, and wherein the side outlet line is connected with the middle section of the dividing-wall distillation column.

9. The plant in accordance with any of the preceding claims, which further comprises i) an overhead condenser and a liquid-liquid separator, wherein the overhead outlet line is connected with the overhead condenser and downstream thereof with the liquid-liquid separator, wherein the liquid-liquid separator is connected with a water line and is connected with a hydrocarbon line, wherein the water line leads into the steam generating unit and the hydrocarbon line splits into a discharge line and into a recycle line being connected with the top section of the dividing-wall distillation column, and/or ii) a side condenser and a liquid-liquid separator, wherein the side outlet line is connected with the side condenser and downstream thereof with the liquid-liquid separator, wherein the liquid-liquid separator is connected with a water line and is connected with a hydrocarbon line, wherein the water line leads into the steam generating unit and the hydrocarbon line splits into a product line and into a recycle line being connected with the second middle subsection of the dividing-wall distillation column.

10. The plant in accordance with any of the preceding claims, which further comprises a reboiler, wherein the bottom section comprises an outlet line and in inlet line, both being connected with a recycle line leading from the outlet line to the inlet line, wherein the recycle line is connected with the reboiler.

11. The plant in accordance with any of the preceding claims, wherein a reboiler is arranged within the bottom section, wherein the dividing-wall distillation column has a circular cross-section and the bottom section comprises an upper subsection having the same diameter than the middle section and a lower subsection having a smaller diameter than the middle section, wherein the reboiler is arranged within the lower subsection of the bottom section.

12. The plant in accordance with any of claims 1 to 10, wherein the subsection of the bottom section, which has a smaller cross-sectional area than the middle section of the dividing-wall distillation column, does not comprise a reboiler and is not connected with a line being connected with a reboiler.

13. The plant in accordance with any of the preceding claims, wherein the steam generating unit comprises a steam generator and a solvent regenerator, wherein the steam generator comprises an inlet being connected with a water line and an outlet for steam, wherein the solvent regenerator comprises an inlet being connected with the outlet for steam of the steam generator, a further inlet line being connected with a solvent line being connected with the bottom outlet line of the dividing-wall distillation column and an outlet being connected with the inlet line for steam of the dividing-wall distillation column.

14. A method for separating and purifying styrene from a crude mixture comprising styrene comprising the steps of:
a) feeding a crude hydrocarbon composition containing styrene via the inlet line for feed, feeding an extractive solvent via the inlet line for extractive solvent and feeding an extractive solvent and water steam comprising mixture via the inlet line for steam into the dividing-wall distillation column of a plant in accordance with any of the preceding claims,
b) distilling the crude hydrocarbon composition, the extractive solvent and the water steam in the divided-wall column,
c) withdrawing a pure styrene composition via the side outlet line of the dividing-wall distillation column.

15. The method in accordance with claim 14, wherein the crude hydrocarbon composition contains 10 to 80% by weight, preferably 30 to 60% by weight and more preferably 40 to 50% by weight of styrene, wherein the crude hydrocarbon composition is preferably selectivelyhydrotreated hydrocarbon C8 cut, wherein the extractive solvent is a sulfone, preferably a cyclic or acyclic sulfone and more preferably sulfolane.
